# EUROPEAN PATENT APPLICATION

(11) **EP 4 431 595 A1**
(43) Date of publication of application: **18.09.2024**
(21) Application number: 22892921.2
(22) Date of filing: 14.11.2022
(51) Int. Cl.: C12N 5/07

(54) **LONG-TERM CULTURE OF LARGE INTESTINE EPITHELIAL CELLS**

(30) Priority: 12.11.2021 JP 2021184912
(71) Applicant: ISM Co., Ltd., Tokyo 1020082 (JP); Ochiya, Takahiro, Tokyo 1040053 (JP); Miyato, Mitsuru, Kanagawa 2510052 (JP)
(72) Inventor: OCHIYA, Takahiro, Tokyo 104-0053 (JP); MIYATO, Mitsuru, Fujisawa-Shi, Kanagawa 251-0052 (JP)
(74) Representative: D Young & Co LLP
(86) International application number: PCT/JP2022/042210
(87) International publication number: WO 2023/085423

(57) **Abstract**

The present invention relates to a method for long-term culture of colorectal epithelial cells, the method comprising culturing colorectal epithelial cells in the presence of a ROCK inhibitor.

## Description

### Technical Field

The present invention relates to a method for preparing extracellular vesicles derived from colorectal epithelial cells and a method for long-term culture of colorectal epithelial cells.

### Background Art

It has been reported that Rho-associated kinase (ROCK) inhibitors contribute to survival of human embryonic stem cells (Non Patent Literature 1). Reports on actions of ROCK in culture of adult stem cells have shown that epithelial stem cells can be proliferated in vitro for a long period by inhibiting PAK1-ROCK-Myosin II and TGF-β signaling (Non Patent Literature 2), that inhibition of ROCK and regulation of extracellular Ca²⁺ concentrations enable growth of epithelial cells of the nasal mucosa (Non Patent Literature 3), and that adult stem cells derived from amnion epithelial cells are cultured in the presence of a ROCK inhibitor (Patent Literature 1).

### Citation List

### Patent Literature

Patent Literature 1
International Publication No. WO 2009/061024

### Non Patent Literature

Non Patent Literature 1
   Nat. Biotechnol. 25, 681-686 (2007)
Non Patent Literature 2
   Cell Reports 25, 598-610 (2018)
Non Patent Literature 3
   Sci. Rep. 10, 16853 (2020)

### Summary of Invention

### Technical Problem

However, long-term culture of colorectal epithelial cells has been difficult heretofore. Therefore, when a study is conducted using cells in vitro to investigate disorders and diseases caused by abnormal colorectal epithelial cells, it has been necessary to prepare cells for each study. There has also been a problem that long-term effects and actions of a pharmaceutical agent on colorectal epithelial cells cannot be investigated.

### Solution to Problem

The present inventors have explored methods for long-term culture of colorectal epithelial cells to find that culturing colorectal epithelial cells in the presence of a ROCK inhibitor allows the long-term culture thereof, and thus accomplished the present invention.

### Advantageous Effect of Invention

The method of the present invention allows long-term culture of colorectal epithelial cells.

### Brief Description of Drawings

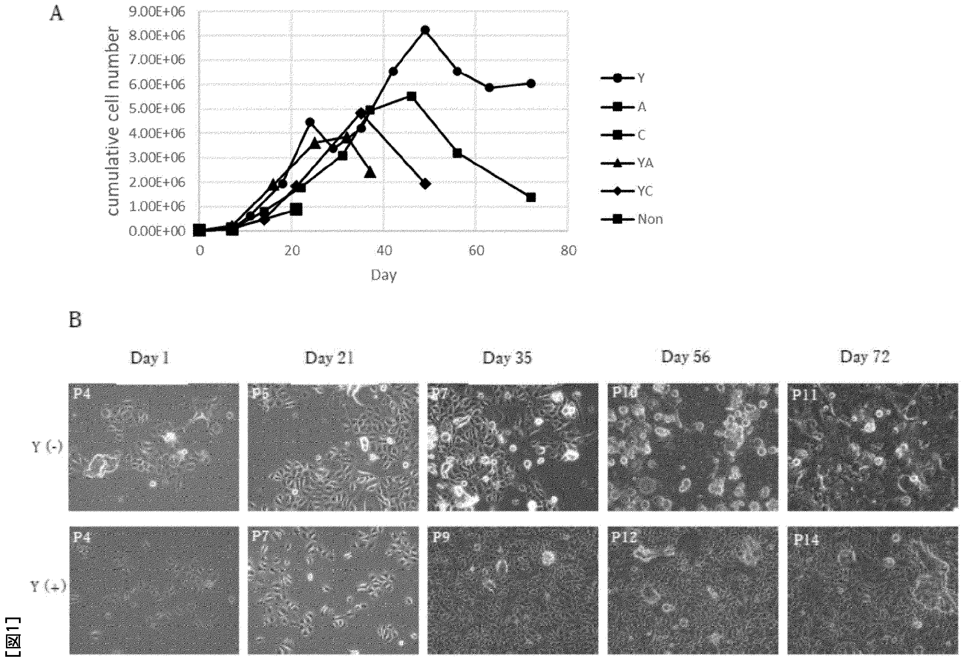

[Figure 1] Figure 1(A) shows a graph showing the number of cells during cell culture in the presence of each test compound. The vertical axis represents the number of cells. Long-term culture was enabled in the presence of Y, and no morphological change was observed. Figure 1(B) shows photographs of cells in the absence of a pharmaceutical agent (Y(-)) and in the presence of Y (Y(+)) at Day 1, Day 21, Day 35, Day 56, and Day 72 from the start of cell culture.

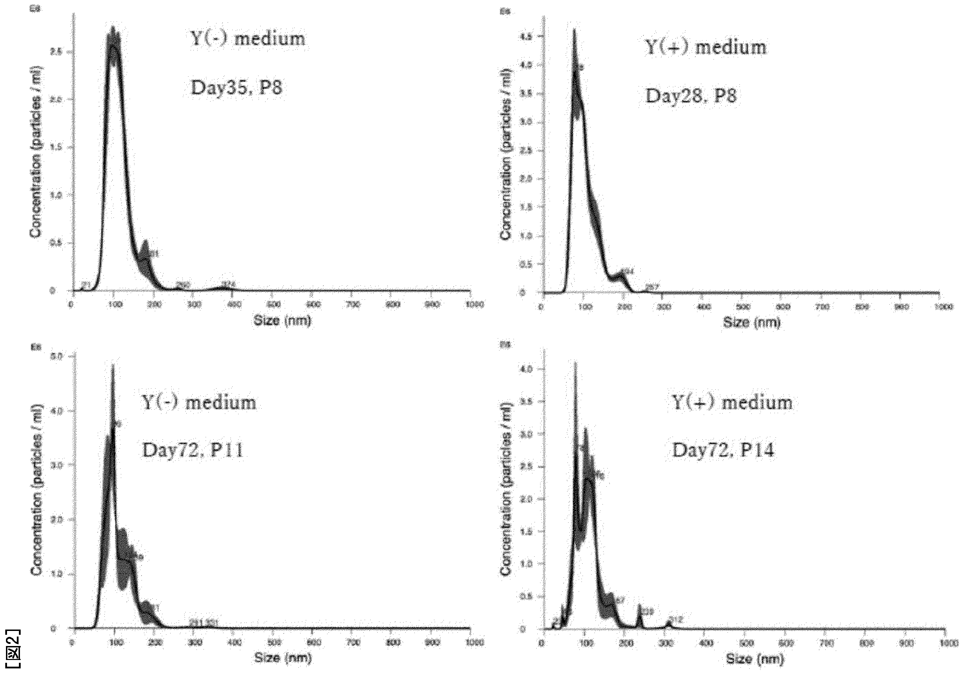

[Figure 2] Figure 2 shows graphs showing particle number concentrations (particles/mL) in a culture supernatants at Day 28, Day 35, and Day 72 from the start of cell culture when cells were cultured in the absence of the pharmaceutical agent (Y(-)) and in the presence of Y (Y(+)). The vertical axis represents the particle concentration (particles/mL), and the horizontal axis represents the particle diameter (nm). "P" denotes a passage, and the subsequent number is a passage number of cells. The particle diameter of released vesicles was unchanged in the presence of Y.

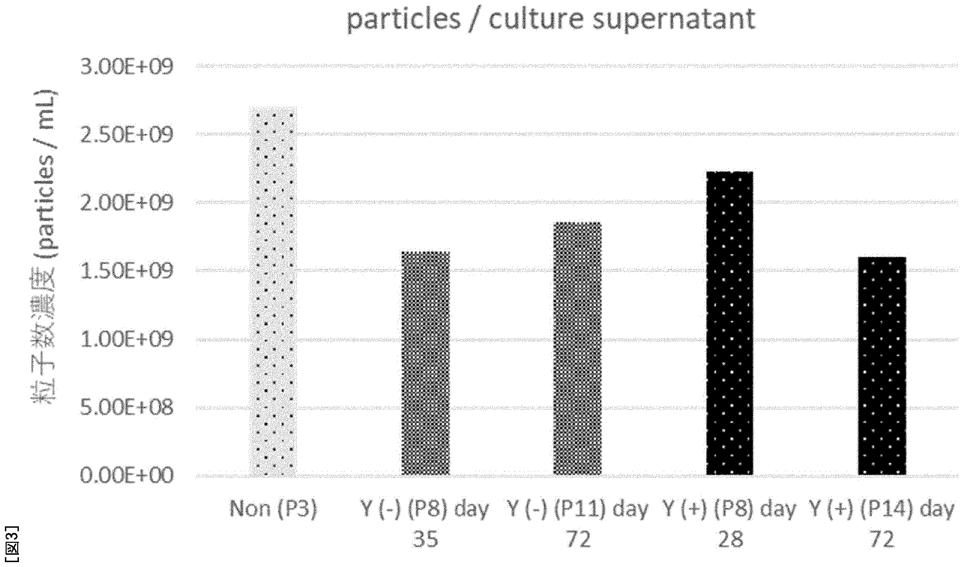

[Figure 3] Figure 3 shows a graph showing particle number concentrations (particles/mL) in a culture supernatants at Day 28, Day 35, and Day 72 from the start of cell culture when cells were cultured in the absence of a pharmaceutical agent (Y(-)) and in the presence of Y (Y(+)). "P" denotes a passage, and the subsequent number is a passage number of cells.

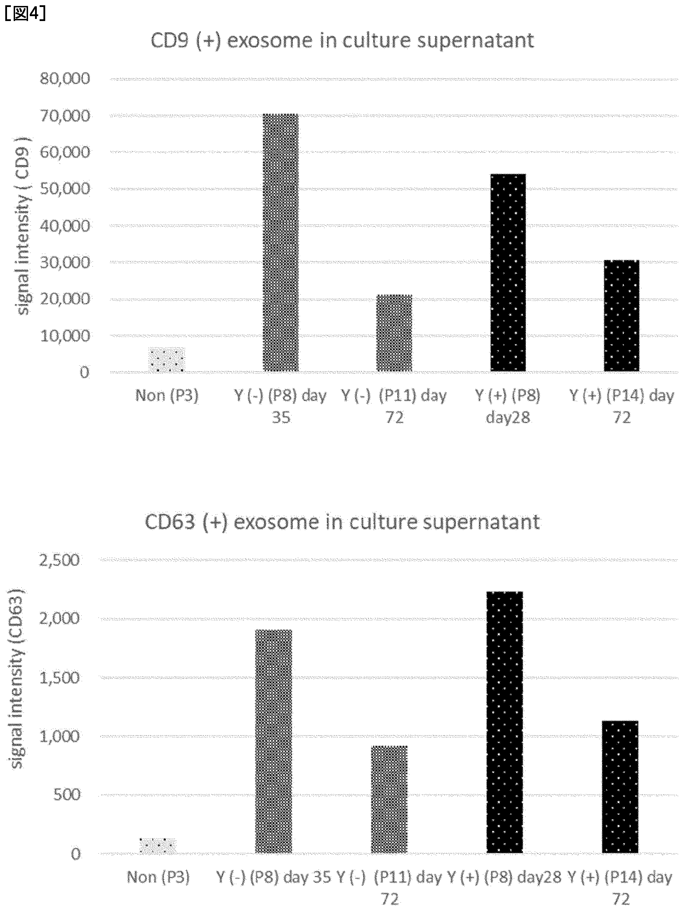

[Figure 4] Figure 4 shows graphs showing signal intensity based on CD9(+) exosome and CD63(+) exosome in culture supernatants. The vertical axis represents the signal intensity. "P" denotes a passage, and the subsequent number is a passage number of cells.

### Description of Embodiments

An intestinal epithelium is a dynamic tissue of which regeneration is repeated every 3 or 4 days. A colorectal epithelial cell is defined as a cell differentiated from a Lgr5-positive intestinal epithelial stem cell, which has permanent self-renewal capacity and differentiation capacity into any intestinal epithelial cell and serves as driving forces of the intestinal epithelium. Intestinal epithelial cells are primarily classified into four types of intestinal epithelial cells: absorptive epithelial cells, goblet cells, Paneth cells, and endocrine cells. Among these, intestinal epithelial cells which are present in the large intestines, in particular, can be used as colorectal epithelial cells in the present invention. Intestinal epithelial cells, preferably colorectal epithelial cells used in the present invention, characteristically express epithelial cell marker molecules, such as E-cadherin. Preferably, colorectal epithelial cells can process an antigen to present it to T cells and/or express HLA class II and intercellular adhesion molecules in vivo in response to a stimulus. The colorectal epithelial cells may be characterized by expressing α3, α5, α6, β1, and β4 integrins. Further, either a primary cultured cell or a cell line may be used as a colorectal epithelial cell in the present invention.

In the present specification, a "ROCK inhibitor" refers to a pharmaceutical agent which directly binds to Rho-associated kinase (ROCK) to inhibit functions thereof. Rho-associated kinase is a serine-threonine phosphorylase with a molecular weight of approximately 160 kDa and has isoforms, Rho kinase α/ROKα/ROCK2 and Rho kinase β/ROKβ/ROCK1. ROCK in the present specification may be either or both of these isoforms. ROCK inhibitors may be ROCK2 inhibitors, ROCK1 inhibitors, or dual inhibitors of ROCK1 and ROCK2, and examples thereof include the following:
4-[(1R)-1-aminoethyl]-N-pyridin-4-ylcyclohexane-1-carboxamide (Y-27632),
5-(1,4-diazepane-1-sulfonyl)isoquinoline (fasudil hydrochloride (HA-1077 hydrochloride)), N-benzyl-2-(pyrimidin-4-ylamino)thiazole-4-carboxamide (thiazovivin),
N-(6-fluoro-1H-indazol-5-yl)-2-methyl-6-oxo-4-(4-(trifluoromethyl)phenyl)-1,4,5,6-tetrahydropyridine-3-carboxamide (GSK429286A),
N-[(3-hydroxyphenyl)methyl]-N'-[4-(4-pyridinyl)-2-thiazolyl]-urea (RKI-1447),
(S)-4-methyl-5-[[2-methyl-1,4-diazepan-1-yl]sulfonyl]-Isoquinoline dihydrochloride (H-1152 dihydrochloride), and
6-chloro-N⁴-[3,5-difluoro-4-[(3-methyl-1H-pyrrolo[2,3-b]pyridin-4-yl)oxy]phenyl]-2,4-pyrimidinediamine (azaindole 1 (TC-S 7001)).

In the present specification, the term "extracellular vesicles" is a collective term for vesicles released from cells and includes ectosomes, microparticles, shedding microvesicles, exosomes, microvesicles (MVs), and apoptotic bodies.

An "exosome" is an extracellular vesicle released from various cells via an endocytosis pathway and contains proteins and/or nucleic acids (mRNA, miRNA, noncoding RNA). Exosomes have various intercellular communication functions including intercellular communication, antigen presentation, and transportation of proteins and nucleic acids such as mRNA and miRNA, and are usually extracellular vesicles with a diameter of approximately 20 to 200 nm or 50 to 150 nm. Alix, Tsg101, tetraspanins (CD81, CD63, CD9), and flotillin are known as exosome markers, and exosomes preferably have CD9 and CD63 on the surface thereof.

In one embodiment, the present invention relates to a method for long-term culture of colorectal epithelial cells, the method comprising culturing colorectal epithelial cells in the presence of a ROCK inhibitor.

Culture of colorectal epithelial cells is not particularly limited, as long as the culture is performed in a medium containing a ROCK inhibitor. For example, colorectal epithelial cells can be cultured by culturing colorectal epithelial cells at 37°C in a 5% CO₂ environment.

The method of the present invention allows long-term culture of colorectal epithelial cells.

As described below, the method of the present invention allows long-term culture of colorectal epithelial cells and recovery of exosomes or extracellular vesicles from proliferated cells, and can also investigate effects and actions on colorectal epithelial cells over a long period.

In the present invention, a ROCK inhibitor is not particularly limited, but Y-27632 is preferably used as described in the examples.

Culture in the method of the present invention can also be performed in the presence of substances other than a ROCK inhibitor which can act on colorectal epithelial cells and enable long-term culture, or substantially in the absence of substances other than a ROCK inhibitor which act on colorectal epithelial cells and enable long-term culture. In the present invention, culture may be performed, for example, in the absence of selective ALK5/7/4 inhibitor and/or GSK-3 inhibitor.

A medium used for culture is not particularly limited as long as the medium is suitable for culturing colorectal epithelial cells, and examples thereof include a colonic epithelial cell medium (CoEpiCM, SCR).

Additives added to the medium are not particularly limited, and examples thereof include a colonic epithelial cell growth supplement (CoEpiCGS, SCR) and/or antibiotics (e.g., penicillin/streptomycin), .

During culture in the present invention, colorectal epithelial cells may be cultured in a complete medium as detailed in the examples.

In the present invention, colorectal epithelial cells are cultured, preferably, by supporting cells on the coated surface of an incubator. Such coatings are not particularly limited, and examples thereof include biological coatings (coating with ECM protein) and chemical coatings (coating with polylysine).

The period for long-term culture can be 10 days or longer, 20 days or longer, 21 days or longer, 30 days or longer, 36 days or longer, 40 days or longer, 45 days or longer, 56 days or longer, 60 days or longer, 70 days or longer, 72 days or longer, or 80 days or longer. Further, the period for long-term culture may be 14 days or longer, 21 days or longer, 28 days or longer, 35 days or longer, 42 days or longer, 49 days or longer, 56 days or longer, or 72 days or longer. The period for the culture may be 28 days or longer or may be 35 days or longer, preferably 40 days or longer. A preferred period of 40 days or longer may be 45 days or longer, 56 days or longer, 60 days or longer, 70 days or longer, 72 days or longer, 80 days or longer, or may be 42 days or longer, 49 days or longer, 56 days or longer, 72 days or longer.

In another embodiment, the present invention relates to a method for preparing extracellular vesicles derived from colorectal epithelial cells, the method comprising: culturing colorectal epithelial cells in the presence of a ROCK inhibitor; recovering a culture supernatant after the culture; and recovering extracellular vesicles from the culture supernatant.

Culture of colorectal epithelial cells in this method can be performed in accordance with the above-described method for long-term culture of colorectal epithelial cells.

Extracellular vesicles or exosomes can be recovered by an arbitrary known method using a culture supernatant after completion of culture. For example, a method used to recover extracellular vesicles from a sample is not particularly limited, and examples thereof include ultracentrifugation (e.g., Thery C., Curr. Protoc. Cell Biol. (2006) Chapter 3: Unit 3.22), polymer precipitation, immune sedimentation, FACS, ultrafiltration, gel filtration, HPLC, and a method of adsorbing extracellular vesicles to a carrier such as a bead using an antibody or lectin. Extracellular vesicles or exosomes may also be recovered using a commercially available kit.

When an antibody is used to recover extracellular vesicles, for example, exosomes, they may be recovered using a carrier bound with an anti-CD9 antibody and an anti-CD63 antibody to utilize CD9 and CD63 on the exosome surface.

A step of recovering exosomes may comprise, for example, mixing a culture supernatant and a carrier bound with an anti-CD9 antibody and an anti-CD63 antibody and recovering the carrier bound with exosomes. This step may further comprise a step of washing the carrier bound with exosomes, a step of dissociating exosomes from the carrier, and the like.

When ultracentrifugation is used to recover extracellular vesicles, the centrifugal force is not particularly limited, and for example, centrifugation may be performed at 50,000 × g or higher, 100,000 × g or higher, or 150,000 × g or higher, and/or 300,000 × g or lower, 250,000 × g or lower, or 200,000 × g or lower. The centrifugation time is not particularly limited and can be, for example, 30 to 120 minutes, 60 to 90 minutes, or 70 to 80 minutes. Additionally, filter filtration and/or centrifugation at a lower centrifugal force can be performed as needed before centrifugation to remove or reduce impurities.

Recovery of extracellular vesicles or exosomes or physical properties of exosomes can be confirmed by a known method. For example, these may be confirmed visually using an electronic microscope, or the particle diameter and the particle number of exosomes may be measured using a nano tracking analysis (NTA) technique. Alternatively, the existence of exosomes may be confirmed by confirming expression of a protein and/or a gene which can serve as an exosome marker.

A sample used for preparation of extracellular vesicles or exosomes is not particularly limited as long as it is a culture supernatant of colorectal epithelial cells cultured in the presence of a ROCK inhibitor, but preferably, it is a culture supernatant of colorectal epithelial cells cultured for 72 days or longer. The culture supernatant of colorectal epithelial cells may be a culture supernatant of colorectal epithelial cells cultured for 10 days or longer, 20 days or longer, 21 days or longer, 30 days or longer, 36 days or longer, 40 days or longer, 45 days or longer, 56 days or longer, 60 days or longer, 70 days or longer, 72 days or longer, or 80 days or longer, or may be a culture supernatant of colorectal epithelial cells cultured for 14 days or longer, 21 days or longer, 28 days or longer, 35 days or longer, 42 days or longer, 49 days or longer, 56 days or longer, 72 days or longer. The culture period for colorectal epithelial cells to obtain a culture supernatant may be 28 days or longer, or may be 35 days or longer, preferably 40 days or longer. The preferred 40 days or longer may be 45 days or longer, 56 days or longer, 60 days or longer, 70 days or longer, 72 days or longer, or 80 days or longer, or may be 42 days or longer, 49 days or longer, 56 days or longer, or 72 days or longer.

The culture supernatant can be recovered by a conventional known method.

### Examples

The present invention will be described in more detail using the following examples below, but the scope of the present invention is in no way limited to these examples. It should be noted that the contents of all documents cited throughout the present specification are hereby incorporated by reference in their entirety.

### (Example 1) Effect of a ROCK inhibitor on release of exosomes from colorectal epithelial cells

### (1) Materials

### (Cells)

Regarding Primary human colonic epithelial cells (HCoEpiC), Lot No. 17030 was purchased from ScienCell Research Laboratories, Inc. (SCR, Carlsbad, CA, United States) and used.

### (Medium)

A complete medium containing 5 mL of a colonic epithelial cell growth supplement (CoEpiCGS, SCR) and 5 mL of penicillin/streptomycin (P/S, SCR) added to 500 mL of a colonic epithelial cell medium (CoEpiCM, SCR) as a basal medium was used as a medium.

### (Low molecular weight compounds)

To investigate the effect on release of exosomes, 10 µM Y-27632 (ROCK inhibitor) (Wako), 0.5 µM A-83-01 (ALK5/7/4 selective inhibitor) (Wako), and 3 µM CHIR99021 (GSK-3 inhibitor) (Axon Medchem, Reston, VA) were used.

### (2) Culture

Cells were completely dissolved in a water bath at 37°C and seeded on 10 mL of the complete medium in a 10-cm dish coated with poly-L-lysine (poly-Llysine 2 µg/cm², SCR) at 2.5 × 10⁵ cells/dish. On the following day, the medium was replaced with a fresh complete medium. The medium was replaced with a complete medium every 3 or 4 days until 70% confluency was reached and then every other day while 70% to 90% confluency was reached.

Cells reaching 90% confluency were removed using a 0.05% trypsin/EDTA solution (T/E, SCR) and seeded onto a 12-well plate (Fisher-brand 12-well tissue culture plate with a flat bottom) at 2 × 10⁴ cells/well. Twenty four hours later, the medium was replaced with a complete medium containing or not containing Y-27632 (10 µM), and thereafter the medium was replaced every other day until 90% confluency was reached. When 90% confluency was reached, the cells were subcultured again, and the above-described procedures were continued for 72 days.

### (3) Analysis of particles

The culture supernatant in the culture was recovered, and the particle diameter and the particle number were measured using a nanoparticle analysis system NanoSight (NS300, Malvern Panalytical Ltd.). Then, the signal intensities of exosome markers, CD9 and CD63, in the recovered culture supernatant were measured by an ExoScreen assay.

### (4) Results

### (Maintenance of epithelial morphology and prolongation of survival time by low molecular weight compounds)

Among HCoEpiC cultured in the presence of a low molecular weight compound, Y-27632 (Y), A-83-01 (A), or CHIR99021 (C), a combination of Y-27632 and A-83-01 (YA), or a combination of Y-27632 and CHIR99021 (YC), continued growth over the longest period was shown in a group using the medium to which Y-27632 was solely added (Figure 1A). In the group using the medium to which Y-27632 was solely added, the morphology was unchanged after 72 days of culture, confirming continued growth (Figure 1B).

Specifically, it was confirmed that culturing colorectal epithelial cells in the presence of a ROCK inhibitor, (1R,4r)-4-((R)-1-aminoethyl)-N-(pyridin-4-yl)cyclohexanecarboxamide (Y-27632) allowed long-term culture of colorectal epithelial cells.

### (Secretion of exosomes from HCoEpiC cultured in Y-27632-added medium)

Measurement of the particle number concentration using NanoSight showed that the particle number concentration in the medium at Day 72 of culture was higher in the absence of Y-27632, and the ExoScreen assay using CD9 and CD63 markers showed that more exosomes were detected in a culture supernatant of HCoEpiC cultured in the presence of Y-27632 (Figure 4).

Further, it was confirmed that CD9(+) exosomes decreased only by about 1/3 after repeated passages in the presence of Y-27632, but decreased by 2/3 or more in the absence of Y-27632.

## Claims

1. A method for long-term culture of colorectal epithelial cells, the method comprising
culturing colorectal epithelial cells in the presence of a ROCK inhibitor.

2. The method according to claim 1, wherein the ROCK inhibitor is (1R,4r)-4-((R)-1-aminoethyl)-N-(pyridin-4-yl)cyclohexanecarboxamide.

3. The method according to claim 1 or 2, wherein a period for the culture is 40 days or longer.

4. A method for preparing extracellular vesicles derived from colorectal epithelial cells, the method comprising:
culturing colorectal epithelial cells in the presence of a ROCK inhibitor;
recovering a culture supernatant after the culture; and
recovering extracellular vesicles from the culture supernatant.

5. The method according to claim 4, wherein the ROCK inhibitor is (1R,4r)-4-((R)-1-aminoethyl)-N-(pyridin-4-yl)cyclohexanecarboxamide.

6. The method according to claim 4, wherein the extracellular vesicles are exosomes.

7. The method according to any one of claims 4 to 6, wherein a period for the culture is 28 days or longer.
